# EUROPEAN PATENT APPLICATION

(11) **EP 4 122 944 A1**
(43) Date of publication of application: **25.01.2023**
(21) Application number: 21187015.9
(22) Date of filing: 21.07.2021
(51) Int. Cl.: C07K 7/64, C12N 9/48, C12P 21/02

(54) **ENZYMATIC SYNTHESIS OF CYCLIC PEPTIDES**

(71) Applicant: EnzyTag B.V., 6361 HK Nuth (NL)
(72) Inventor: VAN DEN BOS, Leendert Johannes, 6361 HK Nuth (NL); BAIERL, Anna Christina, 6361 HK Nuth (NL); VAN DUN, Sam, 6361 HK Nuth (NL); PLAUM, Maarten Elisabeth, 6361 HK Nuth (NL)
(74) Representative: V.O.

(57) **Abstract**

The invention relates to a method for enzymatically synthesising a cyclic peptide, comprising providing a precursor peptide, wherein the precursor peptide comprises a first reactive group, which first reactive group is an unprotected amine group and a second reactive group which second reactive group is a peptide ester or peptide thioester, wherein at least one of said reactive groups is a reactive side-chain or a part of a side-chain of the precursor peptide,
and subjecting the precursor peptide to an enzymatic cyclization in the presence of a peptide cyclase, wherein the first reactive group and the second reactive group form an amide bond whereby the cyclic peptide is obtained, with the proviso that at least one of said reactive groups is the reactive side-chain or part of a side-chain of the precursor peptide.

## Description

The invention relates to a method for enzymatically synthesizing cyclic peptides.

As observed in WO2010/060112, polypeptides have important applications as pharmaceuticals. Polypeptides, such as short peptides, however, often suffer from poor metabolic stability, poor cell penetrability, and promiscuous binding due to conformational flexibility. Various approaches to stabilizing helical peptides have been tried, for example by using intramolecular crosslinkers to maintain the peptide in a desired configuration by introducing disulphide bonds, amide bonds, or carbon-carbon bonds to link amino acid side chains. In WO2010/060112 it is acknowledged that there remains a significant need for therapeutic and pharmaceutically useful polypeptides with improved biological properties such as improved in vivo half-lives, efficacy at lower doses or reduced frequency of administration. WO2010/060112 aimed to contribute to this need by providing helical peptidomimetic macrocycles with improved pharmacokinetic properties relative to their corresponding non cross-linked counterparts.

WO 2005/3414 is an example of a publication wherein a cyclic peptide is described having two cyclic structures. At least one of said cyclic structures is formed via bonding the N-terminal amine (first reactive group) and the C-terminal carboxylic acid function (second reactive group). The other cyclic structure can be formed via an amide bond or via a disulphide bond or a methylene bridge between two sulphur atoms of two different amino acids.

Biron et al., 'Chapter 9 : Synthetic Strategies for Macrocyclic Peptides' in 'Practical Medicinal Chemistry with Marcrocycles: Desgin, Synthesis, and Case Studies, Wiley Online Library, Eric Marsault and Mark L. Peterson (editors), 18 August 2017 (e.g. available via doi.org/10.1002/9781119092599.ch9), discusses in more detail various possibilities for synthesising cyclic peptides (peptide macrocycles). Biron et al. discuss that although biological methods, such as phage display, can produce cyclic peptides in very large numbers, chemical synthesis remains the method of choice to generate structural diversity, optimize pharmacological properties and to obtain sufficient quantities for biological investigations. Various chemical synthesis options are discussed in detail with factors to consider during chemical synthesis design, including a discussion of four general possibilities to form the cyclic structure from a peptide to be subjected to cyclisation: from N-terminus head to C-terminus tail (head-to-tail), from N-terminus head to C-terminus tail (head-to-side chain), from side-chain to C-terminus tail (side chain-to-tail) and from side-chain to side-chain.

Although in some cases the production of macrocycles is straightforward, many are said to be notoriously difficult to prepare. Amongst others, Biron *et al.* discuss that with normal peptide bond formation, ring closing reactions usually proceed much slower and side reactions, such as unwanted intermolecular bond formation can lead to oligomerization and cyclodimerization of the precursor peptide (the peptide from which the cyclic peptide is to be prepared), which side reactions may even predominate. Further, the desired size of the cyclic structure, and thus the length of the peptide chain can be a complicating factor, cyclisation of short peptide chains is often a challenge, but a desired cyclisation of very long peptide chains can also be difficult to accomplish. Moreover, chemical macrocyclization often requires the use of chemical aids (e.g. oxime, triazole, imine, disulphide, metathesis), rather than native amide bond formation (i.e. direct cyclisation by forming an amide bond beween an amine group and a carboxyl group, or its (thio)ester, of a peptide to be cyclised)

In a recent review by Shinbara et al (Frontiers in Chemistry, June 2020, Vol 8, Articicle 447) not only chemical methods of synthesising macrocyclic peptide are discussed but also the possibility to carry out cyclisation from head to tail (backbone cyclisation) by (chemo-)enzymatic cyclisation. E.g. butelase-1 may be used for synthesising macrocycles of 10 or more amino acids, Sortase A can be used for the synthesis of macrocycles of 16 or more amino acids, and Omniligase-1 can be used for backbone cyclisation of linear peptides to form macrocycles of 13 or more amino acids.

Thus, as illustrated by the above cited documents, cyclic peptides form a highly interesting class of bioactive compounds, showing a high molecular diversity and having a wide spectrum of activity that may e.g. be used for various pharmaceutical applications or as a research or development tool, e.g. in the search for pharmaceutically useful polypeptides with improved biological properties such as improved in vivo half-lives, efficacy at lower doses, reduced frequency of administration or which may be suitable for new therapeutic applications. The documents also illustrates that there is an ongoing need for alternative synthesis methodology, because of challenges and limitations in the synthesis with known methods.

It is an object of the present invention to provide such alternative method for synthesising a cyclic peptide. In particular, it is a further object of the invention to provide a method that overcomes at least in part one or more of the drawbacks described in Biron *et al.* or any of the other cited documents. In particular, it is a further object e.g. to provide a method to produce a cyclic peptide, or a library of cyclic peptides, which also allows a convenient method of synthesizing a peptide of interest that is difficult to prepare chemically, that is difficult to isolate in satisfactory purity or yields from the medium wherein it is formed (e.g. chemically or biologically), that is difficult to produce in large quantities, whose known production method is accompanied with undesired side-reactions, e.g. racemisation, or whose known production method may suffer from unwanted side-reactions. E.g. chemical synthesis usually requires considerable protection and subsequent deprotection of potentially reactive amine or carboxylic acid functions of which participation in the cyclization is undesired. In yet a further aspect it is an object to provide a method to efficiently produce a cyclic peptide or a library of cyclic peptides.

One or more other objects that may be subject of the invention follow from the description below.

It has now surprisingly been found possible to synthesise a cyclic peptide by subjecting a peptide to a ring-closure reaction with a first reactive group, which is an amine functional group and a second reactive group, which is an esterified or thio-esterified carboxylic acid functional group of a precursor peptide, wherein at least one of said reactive groups is present at a side-chain of the precursor peptide. Thus, the C-terminus of the precursor peptide, the N-terminus of the precursor peptide or both (in case of a precursor peptide with suitable two side-chains) do not need to participate in the ring closure reaction or both can be used for an additional ring closure, whereby a polycyclic peptide can be formed. Accordingly, the invention relates to a method for enzymatically synthesizing a cyclic peptide, comprising providing a precursor peptide, wherein the precursor peptide comprises a first reactive group, which first reactive group is an unprotected amine group and a second reactive group which second reactive group is a peptide ester or peptide thioester, wherein at least one of said reactive groups is a reactive side-chain or part of a side-chain of the precursor peptide,
and subjecting the precursor peptide to an enzymatic cyclization in the presence of a peptide cyclase, wherein the first reactive group and the second reactive group form an amide bond whereby the cyclic peptide is obtained, with the proviso that at least one of said reactive groups is a reactive side chain or part of a side chain of the peptide.

Such method has been found to be a very suitable alternative to known methods of providing cyclic peptides. In particular, it addresses one or more drawbacks/objects or provides one or more advantages referred to elsewhere herein. For instance, providing a cyclic peptide wherein the C-terminus or the N-terminus of the precursor peptide remains outside the cyclic structure is interesting for various possible reasons. The terminus outside the cyclic structure can for instance be used as a 'handle' for linking the cyclic structure to another moiety, e.g. another biomolecule, a synthetic polymer, a probe (e.g. a fluorescent marker) or a stationary phase material. The latter can for instance be useful for further processing of the reaction product, e.g. for recovery from the reaction mixture wherein the reaction has been carried out or for its purification of the cyclic peptide from the reaction. Attachment to a stationary phase may also be used to immobilise the peptide, e.g. for further development processes or in a drug delivery system.

It is in particular surprising that in accordance with the invention it has become possible to (chemo-)enzymically synthesise peptides comprising a relatively small ring structure, such as a ring structure of 12 amino acid residues or less, even of 10 amino acid residues or less e.g. of 5 or 6 amino acid residues, using an activated carboxyl group of the precursor peptide, i.e. a (thio)ester, as a reactive group in the cyclisation; in terms of covalent bonds defining the ring structure it has even been found possible to enzymatically synthesise a cyclic peptide comprising less than 16 bonds. In contrast, known (chemo-) enzymic methods make use of a head-to-tail cyclisation to form a cyclic structure composed of 12 or more amino acid units. Nonetheless, the present invention can also advantageously be used to synthesise peptides comprising a cyclic structure composed of 12 amino acids or more.

The present invention thereby offers a new tool to synthesise a variety of peptides comprising a cyclic structure, which may be used to create or contribute to a library comprising cyclic peptides.

For the purpose of this invention, with "synthesis over hydrolysis ratio" (S/H ratio) is meant the amount of enzymatically synthesised cyclic peptide product divided by the amount of peptide C-terminal ester or thioester of which the ester or thioester group has been hydrolysed. For further details on determining an S/H ratio, reference is made to WO 2016/056913.

The term "or" as used herein is defined as "and/or" unless it is specified otherwise or it follows from the context that it means 'either ....or...' .

The term "a" or "an" as used herein is defined as "at least one" unless it is specified otherwise or it follows from the context that it should refer to the singular only.

When referring to a noun (e.g. a compound, an additive, etc.) in the singular, the plural is meant to be included, unless it follows from the context that it should refer to the singular only.

The term "pH" is used herein for the apparent pH, i.e. the pH as measured with a standard, calibrated pH electrode.

For the purpose of this invention, with "peptide" is meant any molecular structure at least composed of two or more amino acids joined by an amide bond. Thus, peptides are generally amides at least conceptually composed of two or more amino carboxylic acid molecules *(i.e.* amino acids) by formation of a covalent bond from the carbonyl carbon of one to the nitrogen atom of another with formal loss of water. A moiety in the peptide that at least conceptually is the remains from on amino acids once it has formed the peptide is generally known in the art as an " amino acid residue" or " amino acid unit" of the peptides, although colloquially one may also state that a peptide comprises amino acids, when actually meaning to state a peptide comprises amino acid residues.

The term "peptides" includes peptide conjugates, a conjugate of a peptide of at least two amino acids and another molecule, for instance a carbohydrate (forming a glycopeptide), a nucleotide construct (conjugate of the peptide and a nucleotide, an oligonucleotide or a polynucleotide) or a lipid (forming a lipopeptide) or another moiety, e.g. a stationary phase. Peptides usually comprise a backbone comprising a sequence of two or more alpha-amino acid units, between which a peptide bond is present; however, the backbone of peptides may comprise one or more other amino acids, such as one or more amino acids selected from the group consisting of beta-amino acids, gamma -amino acids, delta-amino acids and epsilon-amino acids. A peptide having a backbone used as a precursor peptide in accordance with the invention generally comprises at least 4 amino acid residues.

Peptides can comprise one or more disulphide bonds (disulphide bridges). Disulphide bonds are typically bonds between two cysteine units (formed by oxidation). Thus, two amino acids in a same peptide chain (amino acid sequence) can be covalently bound, also if they are not adjacent amino acids in the amino acid sequence. Also, a disulphide bond between a first cysteine of a first peptide chain and a second cysteine of a second peptide chain, which may have the same or a different amino acid sequence, can be formed to form a peptide. Such peptide comprises more than one peptide chain. An example of a peptide composed of more than one peptide chain, wherein the different chains are bound via a disulphide bond is insulin. Other bonds to join different peptide chains are generally known in the art.

Typically, peptides - which term includes oligopeptides, proteins and chimeric peptides - comprise up to about 35 000 amino acid units, in particular. 3-20 000, more in particular 4-1000 or 5-500 amino acid units.

For the purpose of this invention, with "peptide bond" is meant the amide bond between an amine group of a first amino acid and a carboxyl group of a second amino acid. The peptide bond can be a bond between the alpha -amino terminus of a first alpha-amino acid and the alpha-carboxyl terminus of a second alpha-amino acid (also referred to in the art as an eupeptidic bond) or an isopeptidic bond, i.e. a peptide bond formed between an amine group of a first amino acid and a carboxyl group of a second amino acid, wherein at least one of said groups is not the alpha-group. E.g. a side chain of a peptide is generally bound to the backbone via an isopeptidic bond.

A "cyclic peptide" is a peptide comprising a cyclic structure comprising a sequence of a plurality of amino acid units bound via a peptide bond. Such a cyclic structure is also referred to in the art as a 'macrocycle' or 'macrocyclic structure'. Not all units in the cyclic structure need to be amino acid units. Not all bonds need to be peptide bonds. A cyclic peptide synthesised in accordance with the invention is generally not a homodetic peptide. At least one of the bonds of the cyclic sequence defining the cyclic structure of a cyclic peptide synthesized in accordance with the invention is typically an isopeptidic bond; typically at least the peptide bond linking the backbone to a side-chain providing a reactive group that has participated in the cyclization; alternatively or in addition an isopeptidic bond is formed between a reactive side group of an amino acid unit in the backbone of the precursor peptide, if such side-group is used for cyclization. Additional isopeptidic bonds can be present in the cyclic sequence, e.g. due to the presence of amino acid units other than alpha-amino acid unit. Thus, a cyclic peptide made in accordance with the invention can be a heterodetic peptide. The cyclic structure can consist of amino acid units. It is also possible to form a cyclic structure of a peptide conjugate; e.g. it can comprise a polyalkylene glycol block, for instance a polyethylene glycol block. It is also possible to include one or more diacids, e.g. C3-C8-diacids e.g. adipic acid or one or more diamines, e.g. C3-C8 diamines in a chain. In a specific embodiment the precursor peptide comprises a proteolysis targeting chimera (PROTAC). As is generally known, a PROTAC is a heterobifunctional molecule capable of removing specific unwanted proteins; PROTACS are typically composed of at least two active domains and a linker. In a method of the invention, a PROTAC can become part of the formed cyclic structure or remain outside the cyclic structure, dependent on the design of the precursor peptide and which amine functionality and which (thio)esterified carboxyl functionality of the precursor peptide are participating in the ring closure reaction.

The cyclic peptide can consist of one or more cyclic structures (if needed after a cleavage of one or more amino acids/peptide chains remaining outside any cyclic structure), but it is also possible to have one or more amino acid units or peptide chains outside the cyclic structure. A cyclic peptide having two or more cyclic structures at least composed of amino acid units, is referred to herein as polycyclic. A bicyclic peptide is a polycyclic peptide having two cyclic structures.

The invention allows the synthesis of a cyclic structure with a surprisingly low number of chemical bonds defining the cyclic structure, although also very large cycles may be created, e.g. of about 1000 bonds or more. Generally, the number of bonds is 10 or more, in particular 12 or more. In practice, a relatively small number of bonds can give constraints, which can make it more of a challenge to achieve satisfactorily effective cyclisation, also dependent on the rigidity of the sequences to define the cyclic structure. In practice, good results are therefore achieved in particular in the synthesis of a cyclic peptide comprising a cyclic structure wherein the number of bonds is 15 or more , in particular 18 or more, 24 or more, or 27 or more. In practice, the number of bonds of the cyclic structure usually is 600 or less, preferably 100 or less, more preferably 60 or less, in particular 45 or less, more in particular about 30 or less. In practice, with an increasing size of the intended cyclic structure, more amino acids may be present in the ring with potentially reactive side-groups contributing to dimerization or other side-reactions. With increasing size, an increased flexibility in the reacting chains may also contribute to a higher risk of side-reactions This can be addressed by protecting potentially reactive groups to avoid them from participating in an enzyme-catalysed reaction if needed). In terms of amino acid units per cyclic structure, it is likewise surprising that a very small cyclic structures can be made, also when the cyclic structure consists of amino acid units. Usually, the cyclic structure synthesised in method according to the invention is composed of at least 5 amino acid units, preferably of at least 6 amino acid units, in particular of least 8 amino acid units. The number of amino acid units in a cyclic structure of the cyclic peptide can e.g. be 200 or more. Usually, the number of amino acid units per cyclic structure is 50 or less, preferably about 30 or less, more preferably 15 or less. Surprisingly good results have also been achieved with a method according to the invention wherein a cyclic peptide structure is obtained having less than 12 amino acid units. Thus in a specifically preferred embodiment the synthesized cyclic peptide, comprises a cyclic structure essentially consists of 4, 5, 6, 7, 8, 9, 10 or 11 amino acid unit.

In the context of the invention with "amino acid" is meant any molecule which is represented by the formula H₂N- R-COOH, wherein R is an organic moiety. The organic moiety usually contains at least one carbon, in particular 1-100 carbons, more in particular 1-50, preferably 1-20 carbons. The organic moiety usually contains at least one hydrogen, in particular 2-200 hydrogens, more in particular 2-100 hydrogens, preferably 2-40 hydrogens. R can consist of carbon and hydrogen atom, e.g. be represent by the formula CₙH₂(ₙ₋ₓ), wherein n is de number of carbon atoms and x the number of unsaturations between two carbons bound to each other, a carbon-carbon double bond counting as a single unsaturation and a carbon-carbon triple bond as two unsaturations). R may be or contain an aryl group, e.g. a phenyl group; R may also contain one or more heteroatoms, in particular one or more heteroatoms selected from the group consisting of S, N, O , P, Cl, F, Br, I, preferably one or more heteroatoms selected from the group consisting of S, N and O. O may in particular be present as part of an ether group, an ester group, an hydroxyl group; N may in particular be present as an amine group (e.g. primary or secondary); S may in particular be present as part of a thiol group, a thioether group or a thioester group; if present, Cl, F, Br or I usually are bound to a carbon (substituting an H). Cl, F, Br or I may e.g. be part of a linker moiety in a side-chain or backbone of the precursor peptide or be part of a PROTAC.

Thus, the term amino acid encompasses both proteinogenic or non-proteinogenic amino acid. Proteinogenic amino acids are the amino acids that are encoded by the genetic code. Proteinogenic amino acids include: alanine (Ala), valine (Val), leucine (Leu), isoleucine (Ile), serine (Ser), threonine (Thr), methionine (Met), cysteine (Cys), asparagine (Asn), glutamine (Gln), tyrosine (Tyr), tryptophan (Trp), glycine (Gly), aspartic acid (Asp), glutamic acid (Glu), histidine (His), lysine (Lys), arginine (Arg), proline (Pro) and phenylalanine (Phe). Selenocysteine (Sec, U) is an amino acid, of which the structure corresponds to cysteine, with the proviso that it contains a selenium instead of a sulphur atom. Proteinogenic amino acids are the L-stereoisomers of said amino acids (except for glycine, which does not have a stereo-isomeric form). Generally, at least a number of the amino acid units forming the cyclic structure are proteinogenic amino acids, in particular from at least half of the amino acid unit to all amino acid units or from at least half of the amino acid unit to all amino acid units minus one or two. Apart from desirable bioactive properties of a sequence comprising proteinogenic amino acids, this can e.g. facilitate the cyclisation e.g. when using a proteinogenic amino acid for one or more of the P1-P4 position or one or more of the P1-P4' position known to be advantageously recognised by a specific cyclase. However, it is also possible to include a non-proteinogenic amino acid at one or more of these positions, even as the amino acid providing a reactive side-chain for the cyclisation or which a side-chain containing a reactive group for the cyclisation.

Non-proteinogenic amino acids of particular interest in a method according to the present invention include beta-amino acids, gamma-amino acids, delta-amino acids, epsilon-amino acids, zeta-amino acids and theta-amino acids. Of specific interest are molecules comprising a polyalkylene glycol chain, wherein one end has an amino group and the other end a carboxyl group, in particular polyalkylene glycols wherein the alkylene is has 2-5 carbons, two carbons.

Of particular interest in a method according to the invention is a precursor peptide comprising a non-proteinogenic amino acid residue having a side-chain amine or carboxylic acid functionality. Such functionality may be able to participate in the cyclization reaction; or a side-chain providing a reactive group for the cyclization can be attached thereto. Preferred non-proteinogenic amino acids are accordingly selected from the group of 2-aminohexane dioic acid, 2-aminoheptanedioic acid, ornithine, 2,4-diamino butanoic acid and 2,3-diamino propionic acid.

However, dependent on the intended amino-acid sequence of the cyclic peptide of interest, it may also be useful to include one or more non-proteinogenic amino acids without an additional amine or carboxylic acid function,. E.g., 2-aminoisobutyric acid (Aib) is an example of a non-proteinogenic amino acid which has found its use in pharmaceutical peptides. Other examples of particularly useful non-proteinogenic amino acid are 5-aminopentanoic acid, gamma-aminobutyric acid and the like.

For the purpose of this invention, with "condensation" is meant the formation of a new peptide bond between a first reactive group (amine) and a second reactive group (ester/thioester) of a peptide.

The term "(thio)ester" is used herein as short-hand for the phrase " ester or thioester".

When an enzyme is mentioned herein with reference to an enzyme class (EC) between brackets, the enzyme class is a class wherein the enzyme is classified or may be classified, on the basis of the Enzyme Nomenclature provided by the Nomenclature Committee of the International Union of Biochemistry and Molecular Biology (NC-IUBMB), which nomenclature may be found at http://www.chem.qmul.ac.uk/iubmb/enzyme/. Other suitable enzymes that have not (yet) been classified in a specified class but may be classified as such, are meant to be included.

The term "mutated", "mutation" or "mutant" as used herein regarding proteins or polypeptides - in particular enzymes such as ligases - means that at least one amino acid in the wild-type or naturally occurring protein or polypeptide sequence has been replaced with a different amino acid, inserted into, appended to, or deleted from the sequence via mutagenesis of nucleic acids encoding these amino acids. Mutagenesis is a well-known method in the art, and includes, for example, site-directed mutagenesis by means of PCR or via oligonucleotide-mediated mutagenesis as described in Sambrook et al., Molecular Cloning-A Laboratory Manual, 2nd ed., Vol. 1-3 (1989). The term "mutated" or "mutation" as used herein regarding genes means that at least one nucleotide in the nucleic acid sequence of that gene or a regulatory sequence thereof, has been replaced with a different nucleotide, has been inserted into, has been appended to, or has been deleted from the sequence via mutagenesis, resulting in the transcription of a protein sequence with a qualitatively of quantitatively altered function or resulting in the knock-out of that gene.

The term "ligase" is used herein for an enzyme having catalytic activity in the coupling of two peptides by catalysing the formation of a peptide bond by coupling the C-terminus of a first peptide and the N-terminus of another peptide.

Cyclases are enzymes capable of forming an amide bond by catalysing a condensation reaction of an amine and a carboxylic acid function. Peptide cyclases are enzymes, which are capable of catalysing the formation of a peptide bond by coupling an amine functional group and a carboxylic acid function (which may be (thio)esterified) of the same peptide, thereby forming a cyclic structure.

Thus, ligases and peptide cyclases catalyse a condensation reaction opposite to a hydrolytic reaction wherein a peptide bond is hydrolysed, which reactions are catalysed by a protease. Accordingly, a ligase or peptide cyclase can be considered to be a protease which catalyses at a higher synthesis rate than hydrolysis rate; S/H of the peptide cyclase is higher than 1, usually > 100, in particular >500, more in particular >1000, e.g. up to 100000, up to 10000 or 5000 or less). The protease can be used in a method according to the invention under conditions that favour the formation of the peptide bond; or a modified protease can be used, wherein, e.g., an active site has been modified to increase the synthesis over hydrolysis ratio. This will be further elaborated herein below.

Schechter and Berger.( Biochem Biophys Res Commun. 1967 Apr 20;27(2):157-62.)) defined that the active site residues in proteases, including ligases (and as the inventors realised: such enzymes having peptide cyclase activity), are composed of contiguous pockets termed subsites. Each subsite pocket binds to a corresponding residue in the peptide substrate sequence, referred to here as the sequence position. According to this definition, amino acid residues in the substrate sequence are consecutively numbered outward from the cleavage sites as...-P4-P3-P2-P1-P1'-P2'-P3'-P4'-...(the scissile bond is located between the P1 and P1' positions), while the subsites (pockets) in the active site are correspondingly labelled as...-S4-S3-S2-S1-S1'-S2'-S3'-S4'. It should be noted that not all proteases have all of said subsites. E.g. an S3' and/or an S4' pocket may be absent. The inventors realised that this not only applies to the N-terminus and C-terminus of peptides to be coupled, but also to a ring closure reaction in a single peptide having one or more side-chains, wherein at least one of the reactive groups is not present at a terminus of the precursor peptide but at a side-chain.

For the purpose of this invention, with "S1, S2, S3 and S4 pocket" is meant the amino acids of a protease (in particular a peptide cyclase) which interact with the amino acids of a peptide acyl donor. The amino acid (1^{st} amino acid; P1) at which the (thio)ester participating in the formation of the peptide bond interacts with the amino acids in the S1 pocket of the protease. The penultimate amino acid (2^{nd} amino acid counted from the (thio)ester; P2) of the acyl donor peptide interacts with the amino acids in the S2 pocket of the protease, the third amino acid (P3) with the S3 and the fourth amino acid (P4) with the S4 pocket. The S1-S4 binding pockets of a protease are defined by several amino acids which can be distant in the primary structure of the protease, but are close in the three dimensional space. For the purpose of this invention, with S1' and S2' pockets are meant the amino acids of a protease (having peptide cyclase activity) which interact with the amino acids near the amine function participating in the formation of the peptide bond. The amino acid (P1') at which the amine participating in the peptide formation is present interacts with the amino acids in the S1' pocket of the protease. The amino acid (P2') adjacent to P1' interacts with the amino acids in the S2' pocket of the protease. The S1' and S2' binding pockets of a protease are defined by several amino acids which can be distant in the primary structure of the protease, but are close in the three dimensional space.

Homologues typically have an intended function in common with the peptide or enzyme, of which it is a homologue, such as being capable of catalyzing the same reaction, in particular an enzymatic coupling of a method according to the invention.

Amino acid or nucleotide sequences are said to be homologous when exhibiting a certain level of similarity. Whether two homologous sequences are closely related or more distantly related is indicated by "percent identity" or "percent similarity", which is high or low respectively.

The terms "homology", "percent homology", "percent identity" or "percent similarity" are used interchangeably herein. For the purpose of this invention, it is defined here that in order to determine the percent identity of two amino acid sequences, the complete sequences are aligned for optimal comparison purposes. In order to optimise the alignment between the two sequences gaps may be introduced in any of the two sequences that are compared. Such alignment is carried out over the full length of the sequences being compared. Alternatively, the alignment may be carried out over a shorter length, for example over about 20, about 50, about 100 or more nucleic acids or amino acids. The percentage identity is the percentage of identical matches between the two sequences over the reported aligned region.

A comparison of sequences and determination of percent identity between two sequences can be accomplished using a mathematical algorithm. The skilled person will be aware of the fact that several different computer programs are available to align two sequences and determine the homology between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley). The percent identity between two amino acid sequences can be determined using the Needleman and Wunsch algorithm for the alignment of two sequences. (Needleman, S. B. and Wunsch, C. D. (1970) J. Mol. Biol. 48, pp 443-453). The Needleman-Wunsch algorithm has been implemented in the computer program NEEDLE. For the purpose of this invention the NEEDLE program from the EMBOSS package was used (version 2.8.0 or higher, EMBOSS: The European Molecular Biology Open Software Suite (2000) Rice,P. Longden,I. and Bleasby,A. Trends in Genetics 16, (6) pp 276—277, http://emboss.bioinformatics.nl/). For protein sequences, EBLOSUM62 is used for the substitution matrix. Other matrices can be specified. The optional parameters used for alignment of amino acid sequences are a gap-open penalty of 10 and a gap extension penalty of 0.5. The skilled person will appreciate that all these different parameters will yield slightly different results but that the overall percentage identity of two sequences is not significantly altered when using different algorithms.

The homology or identity between the two aligned sequences is calculated as follows: the number of corresponding positions in the alignment showing an identical amino acid in both sequences divided by the total length of the alignment after subtraction of the total number of gaps in the alignment. The identity defined as herein can be obtained from NEEDLE by using the NOBRIEF option and is labelled in the output of the program as "longest-identity". For purposes of the invention the level of identity (homology) between two sequences is calculated according to the definition of "longest-identity" as can be carried out by using the program NEEDLE.

The polypeptide sequences, in particular enzyme sequences, can further be used as a "query sequence" to perform a search against sequence databases, for example to identify other family members or related sequences. Such searches can be performed using the BLAST programs. Software for performing BLAST analyses is publicly available through the National Center for Biotechnology Information (http ://www.ncbi.nlm.nih.gov). BLASTP is used for amino acid sequences. The BLAST program uses as defaults:
- Cost to open gap: default = 11 for proteins
- Cost to extend gap: default = 1 for proteins
- Expect value: default = 10
- Wordsize: default = 28 for megablast/ 3 for proteins

Furthermore the degree of local identity (homology) between the amino acid sequence query and the retrieved homologous sequences is determined by the BLAST program. However only those sequence segments are compared that give a match above a certain threshold. Accordingly the program calculates the identity only for these matching segments. Therefore the identity calculated in this way is referred to as local identity.

The term "homologue" is used herein in particular for peptides, more in particular enzymes, having a sequence identity of at least 50 %, preferably at least 60 %, more preferably at least 70 %, at least 80 %, at least 90 %, at least 95 %, at least 96 %, at least 97 %, at least 98 % or at least 99 % with the peptide, in particular enzyme, with which the homologue peptide or enzyme is compared. Evidently, the sequence identity will be less than 100 %. The percentage of sequence identity will depend on the number of mutations and the length of the peptide (enzyme) with which the homologue is compared. In 'longest identity' alignment deletions are not taken into account.

In the context of this application, the term "about" means in particular a deviation of 10 % or less from the given value, more in particular 5 % or less, even more in particular 3 % or less.

When referring to a compound of which stereoisomers exist, the compound may be any of such stereoisomers or a mixture thereof. Thus, when referred to, e.g., an amino acid of which enantiomers exist, the amino acid may be the L-enantiomer, the D-enantiomer or a mixture thereof. In case a natural stereoisomer exists, the compound is preferably a natural stereoisomer.

In the method according to the invention a ring closure is accomplished by forming an amide bond, generally a peptide bond, between an unprotected amine group and a peptide (thio)ester group of the same precursor peptide, wherein the formation of the bond is catalysed by a peptide cyclase. Peptide cyclases are enzymes, which are capable of catalysing the formation of a peptide bond by coupling an amine functional group and a carboxylic acid function of the same peptide, thereby forming a cyclic structure. As discussed above, the enzymatic cyclization of peptides between an unprotected amine group and a peptide (thio)ester group of the same precursor peptide is known in the art from WO2016/056913. However, this publication is directed specifically to cyclization of a peptide (having a length of at least 12 amino acid units) by the formation of a peptide bond by coupling the C-terminus and the N-terminus of the precursor peptide, i.e. a head-to-tail coupling. Thereafter, the use of omniligase-1 has been reported as an enzyme capable of a head-to-tail cyclization, wherein the minimal ring size has to be 11 amino acids (Schmidt et al. Adv. Synthesis & Catalysis, Vol 359, Issue 12, June 2019, 2017 pp2055-2055).

The precursor peptide comprises at least one side-chain comprising a reactive group for the cyclisation. In principle, the side-chain comprising the reactive group can be an amine group (in particular a beta- gamma- delta-, epsilon- or zeta- amino group of an amino acid residue of the precursor peptide) providing the first reactive group or a (thio)ester of a carboxyl group (in particular a beta- gamma- delta-, epsilon- or zeta-carboxyl group of an amino acid residue of the precursor peptide) providing the second reactive group. Usually, the side-chain comprises one amino acid unit comprising one of said reactive groups or a sequence of two or more amino acid units wherein one of said amino units comprises one of said reactive groups. Usually, the reactive group participating in the cyclisation is an amino group or a (thio)esterified carboxyl group of the amino acid residue at the end of the side chain, i.e. the amino acid residue most remote from the backbone, of which amino acid residue either the alpha amino group or the alpha carboxyl group is still present. Typically, such reactive amino group or reactive (thio)esterified carboxyl group of a side chain comprising one or more amino acid units participating in the cyclisation is in the alpha-position. However, in a specific embodiment, the reactive group of a side chain comprising one or more amino acid units participating in the cyclisation can be in another position, in particular in the beta- gamma- delta-, epsilon- or zeta-position.

The precursor peptide can be a natural peptide or a peptide synthesised based on methodology known per se, see e.g. the above cited documents, or the references cited therein. Chemical synthesis can for example be done using solid phase synthesis, which is a generally known technique.

The first reactive group (the amine group) generally acts as a nucleophile in the cyclization reaction in accordance with the invention, which generally can be considered a condensation reaction. The second group is a peptide ester or thioester, typically an activated (thio)ester, i.e. it contains a carboxy ester or carboxy thioester group that can take part in the enzymatic cyclization reaction. The principle of enzymatic coupling of an amine and a (thio)esterified carboxylic acid function to couple peptide fragments or cyclize a peptide are well known in the art for coupling an **N-terminus** of a peptide to the **C-terminus** of a peptide, see for instance US 5,403,737 or WO2016/056913. The choice of the (thio)ester used in accordance with the invention may be based on any of these or common general knowledge.

In principle, any (substituted or unsubstituted) alkyl or (substituted or unsubstituted) aryl (thio)ester can be used. Typical examples of (thio)esters which can take part in the enzymatic coupling reaction are methyl-, ethyl, propyl-, isopropyl-, phenyl-, benzyl- (such as p-carboxy-benzyl-), 2,2,2-trichloroethyl-, 2,2,2-trifluoroethyl-, cyanomethyl- and carboxyamidomethyl-(thio)esters.

Particularly good results have been obtained with carboxyamidomethyl-type esters (Cam-esters) represented by the formula peptide-(C=O)-O-CX₁X₂-C(=O)N-R₁R₂. Herein, each X₁ and X₂ independently represents a hydrogen atom or an alkyl group. Good results have been achieved when both X₁ and X₂ are a hydrogen atom (peptide-(C=O)-O-CH₂-C(=O)N-R₁R₂). Herein R₁ represents a hydrogen atom or an alkyl group and R₂ represents a hydrogen atom or an alkyl group or an amino acid or a peptide residue with a C-terminal carboxyamide or carboxylic acid functionality, optionally protected on the side-chain functionality of the amino acid or on one or more of the side-chain functionalities of the amino acids. Herein, each alkyl group may independently represent a (substituted or unsubstituted) C1-C7 alkyl group, preferably a (substituted or unsubstituted) linear C1-C6 alkyl group, more preferably a (substituted or unsubstituted) linear C1-C3 alkyl group, and most preferably a methyl group. Good results have in particular been achieved in a method of the invention wherein both R₁ and R₂ represent a hydrogen atom or wherein R₁ represents a hydrogen atom and R₂ represents an amino acid or peptide residue with a C-terminal carboxyamide or carboxylic acid functionality, optionally protected on the side-chain functionality of the amino acid or on one or more of the side-chain functionalities of the amino acids.

It is especially advantageous to use a Cam-AA1-AA2 ester or a Cam-AAl ester, wherein AA1 is a first amino acid residue and AA2 is a second amino acid residue. Herein AA1 is a hydrophobic amino acid residue, such as an alanine, valine, leucine, isoleucine, phenylalanine, methionine or tryptophan unit. AA2 is a basic amino acid residue, such as an arginine or a lysine unit. The AA1 and the AA2 may each independently have a free side-chain functionality, i.e. that is free of a protective group or another residue. In particular, good results have been achieved with CamL, i.e. wherein only AA1 is present, and/or AA1 is leucine.

Particularly suitable alternatives for Cam esters are carboxyl substituted benzyl esters, in particular with meta or para-carboxyl substituted benzyl esters represented by the formula peptide-(C=O)-O-CH₂-C₆H₄-CO₂E wherein E represents a hydrogen atom, a positively charged salt ion such as an ammonium ion, or an amino acid or a peptide residue with a *C*-terminal carboxyamide or carboxylic acid functionality, optionally protected on the side-chain functionality of the amino acid or on one or more of the side-chain functionalities of the amino acids. Good results have also been obtained with p-carboxyl substituted benzyl esters represented by the formula peptide-(C=O)-O-CH₂-C₆H₄-CO₂E wherein E is defined as above and in which one or more hydrogen atoms in the phenyl ring (C₆H₄ in the above formula) are replaced by a substituent, such as hydroxy, alkoxy, aryloxy or halogen.

A functional group that should not participate in the cyclization reaction can be protected or unprotected. Whether protection is needed or desirable can be determined empirically in a manner known per se. suitable protection and deprotection methodology is also generally known in the art and may be based on, e.g., WO 2016/056913 or the publications cited therein.

Carboxylic acid groups can for instance be protected with a cyclohexyl, benzyl or allyl group.

Amine functionalities can for instance be protected with an allyloxycarbonyl group or a trifluoroacetyl group.

Further examples of suitable N-protecting groups include carbamate or acyl type protecting groups, for instance 'Cbz' (benzyloxycarbonyl), 'Boc' (tert-butyloxycarbonyl), 'For' (formyl), 'Fmoc' (9-fluorenylmethoxycarbonyl), 'PhAc' (phenacetyl) and 'Ac' (acetyl). The groups For, PhAc and Ac may be introduced and cleaved enzymatically using the enzymes Peptide Deformylase, PenG acylase or Acylase, respectively. Further, a substituted thiocarbamoyl group, such as a phenylthiocarbamoyl (PTC) group is a useful protective group for the N-terminal alpha-amine function. The use of such groups as such is e.g. well known from Edman degradation processes. Protective agents that can be used for Edman-type amino acid sequencing are also referred to herein as Edman-type protective agents, and likewise agents coupled to an amine group (in particular the N-terminal amine) of a peptide are referred to herein as Edman-type protective groups. Suitable protection/deprotection conditions when using Edman-type protective moieties include those that are generally known in the art for using such moiety in Edman-type degradation methods.

The peptide cyclase used can be a peptide ligase with peptide cyclase activity, Suitable peptide cyclases can be selected, e.g., based on the following procedure:
(i) providing a sample of the cyclic peptide in a manner known per se, e.g. by isolation from an organism wherein it is naturally produced, from an genetically modified organism wherein it is produced or by chemical synthesis.
(ii) carrying out a series of experiments wherein in different reaction vessels samples of the cyclic peptide are contacted with different proteases, in particular different serine endoproteases, under hydrolytic conditions.
(iii) identifying which protease has been effective in cleaving a peptide bond in the cyclic structure in the previous step, i.e. wherein a putative precursor peptide for cyclisation has formed
(iv) Identifying the structure of the putative precursor peptide suitable for the cyclisation
(v) Changing reaction conditions to conditions less favourable to hydrolysis whereby the equilibrium between hydrolysis and cyclization shifts towards cyclization or modifying the protease effective in cleaving a peptide bond in the cyclic structure, whereby a peptide ligase is obtained having an increased cyclization over hydrolysis ratio is obtained;
(vi) Verifying that the changed reaction conditions of modification of the protease are effective.

Regarding step (v), the change in reaction conditions can for instance comprise: changing from a reaction mixtures wherein the liquid phase consists of water to a reaction mixture comprising a substantial amount of non-aqueous or even non-protic solvent or even to a reaction mixture that is kept at least substantially free of water, provided that the enzyme retains catalytic activity. One may also keep the ratio of the precursor peptide concentration to the synthesised cyclic peptide concentration high.

In particular a modified protease may be used. The modification is typically a modification in a hydrolytically active site of the protease, whereby the hydrolytic activity is reduced. Good results are for instance achieved with a serine endoprotease, wherein a serine in a hydrolytically active site is substituted by an amino acid reducing the hydrolytic activity to provide the peptide cyclase. Serine proteases can generally be classified in EC 3.4.21. Generally, they have a catalytic triad in the order Asp, His and Ser (). Accordingly, preferably at least one of the amino acids of the catalytic triad has been substituted by another amino acid, such as by a cysteine or a selenocysteine. In particular good results have been achieved with a peptide cyclase, which is a modified serine protease wherein the Ser of the catalytic triad has been replaced by a cysteine.

In particularly good results have been achieved with a subtilisin (EC 3.4.21.62) having peptide cyclase activity, for example thymoligase or omniligase, both available from Enzytag B.V. (Nuth, The Netherlands). Accordingly, a preferred class of enzymes that may be used for the cyclization is the class of subtilisins, subtilisin variants and homologues thereof, having peptide cyclase activity, in particular subtilisin BPN' variants and homologues having peptide cyclase activity. Subtilisins form an enzyme class with considerable importance for their use as detergents. Therefore, subtilisins have been the subject of numerous protein engineering studies. Subtilisins have also been used for the synthesis of oligopeptides, which was, however, almost always accompanied by hydrolytic side-reactions to a significant extent. It was found by Wells et al. (US 5,403,737) that the condensation of oligopeptides in aqueous solution could be significantly improved by altering the active site of subtilisin BPN', a subtilisin from *B. amyloliquefaciens* .When two mutations were introduced, *i.e.* S221C and P225A, a subtilisin BPN' variant called subtiligase was obtained having a 500-fold increased synthesis over hydrolysis ratio (S/H ratio) as compared to wild-type subtilisin BPN'. However, the average ligating yield was around 66% and hydrolysis of the oligopeptide acyl donor C-terminal ester was still substantial. Another drawback of subtiligase was the poor stability against organic cosolvents that are required to solubilize the oligopeptide fragments, against enhanced temperature and against denaturating agents, which are often needed for successful oligopeptide condensation. Therefore, Wells et al. added five additional mutations to subtiligase, i.e. M50F, N76D, N109S, K213R and N218S, to make the enzyme more stable (Proc. Natl. Acad. Sci. USA, 1994, 91, 12544). However, hydrolysis was still a major side reaction. In WO2016/056913 a major improvement is described, accomplished by a deletion of the calcium binding domain, i.e. the positions corresponding to positions 75-83 of subtilisin BPN', in addition to the mutation corresponding to S221 (in the hydrolytically active site of the enzyme). The enzymes described in WO2016/056913 are characterized by a very high synthesis over hydrolysis also in an aqueous environment, albeit that cyclization making use of a side chain providing the amine reactive group and the carboxylic reactive group is not suggested. When referred to subtilisin BPN' in the present disclosure, the wild type sequence as described in WO2016/056913 is meant:
*SUBT_BACAM Subtilisin BPN' Bacillus amyloliquefaciens mature 1 to 275*





 [Sequence ID NO: 1]

In accordance with the present invention, the used peptide cyclase is therefore preferably a peptide cyclase which ,compared to subtilisin BPN', comprises a mutation of the serine at the amino acid position corresponding to S221 of subtilisin BPN' and/or wherein the calcium binding domain has been deleted (i.e. the amino acids corresponding to positions 75-83 of subtilisin BPN'). Such a subtilisin BPN' variant, mutant or homologue is in particular for a high S/H ratio. Further, it has been found that such a subtilisin BPN' variant, mutant or homologue also is capable of catalysing the cyclisation to form a relatively small cyclic structure, such as of a cyclic structure with 30 bonds or less, in particular 20 bonds or less, for instance a ring structure consisting of 12 amino acid residues or less, 9 amino acid residues or less or 5 amino acid residues or less.

Further, the peptide cyclase preferably comprises a mutation of the amino acid corresponding to P225 of subtilisin BPN', selected from the group of amino acid positions corresponding to P225N, P225D, P225S, P225C, P225G, P225A, P225T, P225V, P225I, P225L, P225H and P225Q, more preferably corresponding to P225N, P225D, P225S, P225C, P225G, P225A or P225T. Such mutation is also considered advantageous for a high S/H or for satisfactory cyclisation to obtain a relatively small cyclic structure, e.g. a structure defined by 30 bonds or less, 20 bonds or less, 12 amino acids or less, 9 amino acids or less or 5 amino acids or less.

In addition to said mutation at the position corresponding to P225 or as an alternative, the peptide cyclase preferably comprises 1-16, in particular 6-15, more in particular 12-14 mutations selected from the group of mutations at an amino acid position corresponding to Q2, S3, P5, S9, I31, K43, M50, A73, E156, G166, G169, S188, Q206, N212, T254 and Q271 of subtilisin BPN, wherein 'more preferably one or more of said mutations, more preferably at least six of said mutations, most preferably at least twelve of said mutations are selected from the group of positions corresponding to Q2K, S3C, P5S, S9A, I31L, K43N, M50F, A73L, E156S, G166S, G169A, S188P, Q206C, N212G, T254A and Q271E.

One or more further mutations are advantageously present, at least in addition to the mutation of the position corresponding to S221 (usually into S221C) and the deletion of the calcium binding domain and preferably further in addition to the mutation of P225 and more preferably further in addition to said 1-16 mutations at the positions corresponding to Q2, S3, P5, S9, I31, K43, M50, A73, E156, G166, G169, S188, Q206, N212, T254 and Q271. Such optional mutations are preferably selected from optional mutations mentioned in WO2016/056913 or from mutations mentioned in WO2018/212658, of which the contents are incorporated by reference in particular with respect to the specifics of the described enzymes, more in particular the amino acid mutations mentioned in the claims and the reasons given for the mutations in the description. In particular, compared to subtilisin BPN' *(Sequence ID NO: 1)* the peptide cyclase that used in the present method advantageously, further comprises at least one further mutation selected from the group consisting of amino acid positions corresponding to F189W, F189Y, S33D, S33T, N218D, N218T and N218E. This may be advantageous for improved selectvity, for a high S/H or for satisfactory cyclisation when making a relatively small cyclic structure, e.g. a structure defined by 30 bonds or less, 20 bonds or less, 12 amino acids or less, 9 amino acids or less or 5 amino acids or less.

Particularly preferred with respect to cyclisation effectivity also when forming a relatively small cyclic structure is omniligase, such as omniligase-1 or a homologue thereof. Further, good results have been achieved with thymologase, also to provide a relatively small cyclic structure. Thus, thymoligase or a homologue thereof is a further preferred peptide cyclase used in a method of the invention. Omniligase and thymoligase also have a good S/H ratio and a good stability. Preferably, a peptide cyclase that is a homologue of omniligase respectively thymoligase has at least 90 %, at least at least 95 %, at least 96 %, at least 97 %, at least 98 % or at least 99 % homology (sequence identity) with omniligase-1 respectively thymoligase. In the homologue, at least a S221 mutation and the deletion of the calcium domain is typically conserved. Further, a P225 mutation, such as identified above is preferably present.Furthermore, one or more of the above identified mutations at a position corresponding to Q2, S3, P5, S9, I31, K43, M50, A73, E156, G166, G169, S188, Q206, N212, T254 and Q271 are preferably present.

The precursor peptide which is to be subjected to the enzymatic cyclization in a method according to the invention has at least one side-chain participating in the cyclization reaction. The side chain which may be a reactive side-group directly attached to the backbone (i.e. an amine group or (thio)ester other than an alpha-amine group respectively other than a (thio)ester of an alpha carboxyl). Thus, side-group can in principle be a reactive functionality of an amino acid unit in the backbone. Good results are achieved with a side chain comprising one or more amino acid units, in particular two or more amino acid units, more in particular three or more amino acid units.

In the side chain two or more amino acids are present bound to each other via isopeptide bonds. Thus between two peptide bonds of the side-chain one or more -C-C-bonds can be present whereby the chain length in terms of chemical bonds increases, to increase the ease of interaction of units in the side-chain of which it is desirable that they interact with the cyclase (in particular any unit selected from P1-P4 and P1'-P4').

The side-chain is typically attached to a backbone, which backbone can be a linear peptide chain (the one or more side-chains and the backbone defining a branched peptide having a C-terminus and an N-terminus) or even a cyclic peptide (in which case it has at least two reactive groups each comprising one of the required reactive groups for forming the amide bond whereby the cyclization is realized to form a cyclic peptide having an additional cyclic structure compared to the precursor cyclic peptide). The backbone generally comprises a sequence of alpha-amino acid units, between which a peptide bond is present; however, the backbone may comprise one or more other amino acid residue, such as from one or more amino acids selected from the group consisting of beta-amino acids, gamma-amino acids, delta-amino acids, epsilon-amino acids, zeta-amino acids and theta-amino acids. The backbone may contain a spacer moiety..

In an embodiment, the precursor peptide or synthesized cyclic peptide essentially consists of amino acid units. In a further embodiment, the precursor peptide or synthesized cyclic peptide essentially consists of amino acid units and one or more protective groups. In an embodiment, the precursor peptide or synthesized cyclic peptide is a conjugate of a peptide of two or more amino acids and another molecule, for instance a carbohydrate, (forming a glycopeptide), e.g. a sialic acid moiety or a neutral carbohydrate; a lipid (forming a lipopeptide), e.g. a fatty acid; a nucleotide construct,; a polyalkyene glycol, in particular a polyethylene glycol or a polypropylene glycol; a heterocycle, e.g. piperazine. In a further embodiment, the peptide conjugate is a conjugate of two or more amino acids and an imaging agent, such as a fluorescent, phosphorescent, chromogenic or radioactive group. The peptide conjugate may also contain a chelation agent or toxin.

A side-chain providing the first or the second reactive group participating in the enzymatic cyclisation can in principle be an amine or (thio)esterified carboxyl of any amino acid in a backbone of the precursor peptide, other than an amine or (thio)esterified carboxyl in the alpha-position. A side-chain comprising one or more amino acid units providing the first or the second reactive group participating in the enzymatic cyclisation can in principle be attached to any amine or carboxyl of any amino acid in a backbone of the precursor peptide, other than an amine or carboxyl in the alpha position. The side chain being or comprising the reactive group can for instance be at the same amino acid also providing the other reactive group (i.e. P1 or P1'), the amino acid next to the P1 or P1' amino acid unit, i.e. the P2 or P2' amino acid unit, the amino acid unit next to the P2 or P2' position , i.e. respectively the P3 and P3' position, or the amino next thereto, i.e. the amino acid unit at the P4 or P4' position. A side-chain providing a reactive group for the enzymatic cyclisation may also be at a more remote position from P1. P1-P4 do not even all need to represent amino acids. E.g. cyclisation of a branched peptide having a adipic acid unit at P2 is feasible. As the skilled person will understand, the exact position of the side-chain relative to the other reactive group and length of the side chain will be decided on factors such as the intended structure (sequence of units of which it is to be composed) of the cyclic peptide that is to be prepared.

For a head-to-side-chain cyclisation or tail-to-side-chain cyclisation, the precursor peptide will have either an N-terminus providing the first reactive group or a C-terminus providing the second reactive group. The other reactive group is provided by a side-chain reactive group. Usually, for a head-to-side-chain cyclisation or tail-to-side-chain cyclisation a branched precursor peptide is provided comprising a backbone (with the terminus providing one of the reactive groups) and a side-chain (providing the other reactive group). The reactive group of the side-chain participating in the enzymatic cyclization is either an amine reacting with the peptide C-terminal ester or thioester of the peptide (the alpha C-terminus of the backbone or a (thio)ester reacting with the alpha N-terminal amine (the alpha N-terminus of the backbone) of the peptide (which is unprotected), thereby forming the amide bond. In a specific embodiment, the precursor peptide is a cyclic peptide comprising a side-chain, having either an alpha N-terminus or an alpha C-terminus providing one of the reactive groups, the other reactive group being provided by a side-chain of the cyclic precursor peptide. The resultant cyclic peptide has then one additional cyclic structure, compared to the precursor.

### Tail-to-side-chain

In particular, good results are achieved with a tail-to-side chain cyclisation, wherein the precursor peptide has a peptide C-terminal (thio)ester acting as the second reactive group in the enzymatic cyclisation to form a cyclic peptide structure. For tail-to-side chain a subtilisin BPN' variant, such as described elsewhere herein, is particularly suitable. Thymoligase, omniligase, a thymoligase homologue or an omniligase homoglogue is particularly preferred.

Preferably, the precursor peptide has a side chain comprising one or more amino acid units, and which has an unprotected amine, typically at the end of the side chain , which unprotected amine participates in the enzymatic cyclization as the first reactive group. In principle there is no limit to the length of the side chain, e.g. it may have a length of 50 or more or even 100 or more. However, dependent on the amino acids present in the chain, the risk/tendency of dimerization or other side reactions may increase. Accordingly, preferably, the side-chain has preferably 2-30 amino acid units, more preferably 3-20 amino acid units, in particular 4-16, more in particular 5-14 units, more in particular 5, 6, 7, 8 or 9 amino acid units.

The side-chain providing said first reactive group can be at any position relative to the C-terminal (thio)ester reacting as said second reactive group. It is in particular surprising that the attachment of the side-chain can be at P1, P2, P3 or P4. I.e. , the presence of a branch in P1-P4 does not make it impossible for a peptide cyclase to be capable of catalysing the cyclisation. Being able to provide the side-chain close to the C-terminus is an advantage for the process design, as one can use backbone units at a position that can be relevant for the peptide cyclase activity or selectivity that can remain outside the cyclic structure. Peptide cyclase selectivity or activity can be affected by the backbone unit at the P1, P2, P3 or P4 position. E.g. for a serine endoprotease based peptide ligase, in particular a subtilisin BPN' based peptide cyclase, such as described elsewhere herein,, it can be an advantage to have a hydrophobic amino acid, such as phenyl alanine, valine, isoleucine or leucine, at the P4 position to increase cyclisation activity or selectivity. By having the side-chain for the cyclisation at the P1, P2 or P3 position, an advantageous unit, e.g. for the cyclase activity, selectivity or S/H ratio, at a more remote position relative to the P1 position can be kept out of the final cyclic structure, and e.g. be cleaved off - if desired- from the synthesised cyclic peptide. However, in particular if an advantageous unit at P2, P3 ,P4 is a suitable unit to be present in the cyclic structure of the cyclic peptide to be synthesised, P2 or P2 and P3 or P2, P3 and P4 can advantageously be part of the cyclic structure.

In the side-chain, which provides the amine reactive group, a high flexibility in the sequence of units has been found possible in particular with a peptide cyclase which is a subtilin BPN' variant, such as described elsewhere herein.

In a tail-to-side chain cyclisation, the side chain providing the reactive group is an amine which can be the side-chain amine of an amino acid having at least two amine groups, an amino acid forming a peptide bond with such side-chain amine or a peptide chain bound via a peptide chain with such side-chain amine. The amino acid with at least two amine groups can be a proteinogenic amino acid, i.e. lysine or asparagine. Good results have been achieved with lysine. It is also possible to have a non-proteinogenic amino acid to provide the side-chain amine. The non-proteinogenic amino acid is preferably selected from the group consisting of lysine units, ornithine units, 2,4-diamino butanoic acid units and 2,3-diamino propionic acid units.

Good results have been achieved with a precursor peptide wherein the P4 position comprises a phenylalanine unit. It is contemplated that a hydrophobic amino acid at this position is advantageous for peptide cyclase activity, especially when a subtilisin BPN' variant is used for catalysing the cyclisation. Thus, alternatively, in particular the fourth unit (P4) counted from the C-terminus of the peptide C-terminal ester is selected from the group consisting of valine units, isoleucine units, leucine units, tyrosine units and tryptophan units. Besides, phenylalanine units, valine units, isoleucine units and leucine units are in particular preferred. However, a non-hydrophobic amino acid may alternatively be present with satisfactory results, e.g. a serine.

### Head-to-side chain

In a further advantageous method according to the invention, an enzymatic cyclization is carried out via a head-to-side chain cyclisation. Usually, the precursor peptide from which the cyclic peptide is to be synthesized is a branched peptide, the branched peptide having an unprotected *N*-terminal amine, which unprotected N-terminal amine participates in the enzymatic cyclization as the first reactive group and the branched peptide comprises a side-chain which has the peptide ester or thioester, participating as the second reactive group in the enzymatic cyclization. The reactive (thio)ester is usually at the end of the side-chain, counted from where it is attached to the backbone. For a satisfactory activity, the side-chain for a head-to-side chain cyclisation according to the invention usually comprises one or more amino acid units, i.e. the P1 unit is usually the side-chain or part of the side-chain. The P2 amino acid is usually part of the side-chain or the amino acid at which the side chain is attached to the backbone. Having the P2 as part of the side-chain can have a positive effect on the cyclisation activity, e.g. when comparing cyclisation activity of a branched peptide PAAn-E(SL-OCamL)-AAm, wherein AAₖ and AAₘ -represent the amino acids between the 'branching' amino acid E - at which the side chain sequence ( SL) is attached - and the N-terminus respectively the C-terminus of the backbone, each AA independently representing an amino acid unit and k and m the number of amino acid units in the respective sequences AAₖ and AAₘ. It may be advantageous to have the P2 position outside the side-chain or inside the side-chain, for analogous reasons as given above. It may be advantageous to have the P3 position outside the side-chain or inside the side-chain, for analogous reasons as given above It may be advantageous to have the P4 position outside the side-chain or inside the side-chain, for analogous reasons as given above. If desired, the side-chain may comprise more than four amino acid units. In principle there is no limit to the length of the side chain, e.g. it may have a length of 50 or more or even 100 or more. However, dependent on the amino acids present in the chain, the risk/tendency of dimerization or other side reactions may increase. Accordingly, preferably, the side-chain has preferably 2-30 amino acid units, more preferably 4-20 amino acid units, in particular 6-10 units, more in particular 6, 7, 8 or 9 amino acid units.

In a head-to-side chain cyclisation, the side chain providing the reactive group is a (thio)esterified carbonyl, which can be the side-chain carbonyl of an amino acid having at least two carbonyl groups, an amino acid forming a peptide bond with such side-chain amine or a peptide chain bound via a peptide chain with such side-chain amine. The amino acid with at least two carbonyl groups can be a proteinogenic amino acid, i.e. glutamic acid or aspartic acid. It is also possible to have a non-proteinogenic amino acid to provide the side-chain amine. The non-proteinogenic amino acid is preferably selected from the group consisting of 2-aminohexane dioic acid units and 2-aminoheptanedioic acid units.

In particular, a suitable peptide cyclase may be selected from subtilisin BPN' variants and homologues thereof, such as thymoligase, omniligase, a thymoligase homologue or an omniligase homologue.

At least for peptidic cyclases, such as subtilisin BPN' variants or other proteases with cyclase activity, having S1-S4 and S1'-S4' pockets, a relatively long backbone is considered advantageous for flexibility of the choice of amino acids whilst maintaining a high activity. In particular, a relatively long chain, e.g. providing half the number of amino acids for the cyclic structure or more than half, between the branching amino acid at which the side-chain is present and the N-terminus providing the reactive amine group can be advantageous, considering the flexibility for the peptide nucleophile of subtilisin BPN' variants, such as omniligase or thymoligase or homologues thereof.

### Side-chain-to-side-chain

In an embodiment, the precursor peptides comprises a first side-chain that provide the first reactive group and a second side-chain that provides the second reactive group for carrying out the enzymatic cyclisation. Usually both side chains are composed of one or more amino acids, for steric reasons. Preferably, one or both of said side-chains are composed of two or more amino acid units, in particular of three or more amino acid units, e.g. up to 50 or more. Preferably, one or both of said side-chains are composed of 3-30 amino acid units, more preferably of 4-15 amino acid units, in particular of 5, 6, 7, 8, 9 or 10 amino acid units.

Use may be made of a chain wherein two or more amino acids are present bound to each other via isopeptide bonds, whereby the chain length in terms of chemical bonds increases to overcome or at least reduce steric hindrance.

Further, consideration regarding the side-chains, and the units therein, in particular regarding P1-P4 and Pl'-P4', and the used peptide cyclase, such as a subtilisin BPN' variants or homologue - in particular omniligase or thymoligase or homologues thereof - may be based, mutatis mutandis, on the teaching elsewhere herein.

### Polycyclic peptides

Two conceptual approaches to obtain a cyclic structures are (i) a ring closure by formation of an amide between an amine reactive group and a carboxyl reactive group and (ii) the formation of a bridge between two sulphur groups of two different amino acids in a peptide, such as a disulphide bridge or via a methylene bridge, e.g. as describe in WO 20053414 for a specific peptide.

In the synthesis of a polycyclic peptide, e.g. a bicyclic peptide, according to the invention, at least one of the cyclic structures is accomplished by approach (i) wherein the cyclisation is enzymatically catalysed and at least one of the reactive groups is a side-group or part thereof. This can be a head-to-side chain cyclisation, a tail-to-side chain cyclisation or a side-chain-to-side-chain cyclisation. It is possible that the precursor peptide is already a cyclic peptide, comprising both a side-chain with a reactive amine group and a reactive carbonyl. In such embodiment, a cyclic peptide is provided having a first side chain comprising the first reactive group and a second side chain comprising the second reactive group and these reactive groups are react with each other thereby forming the amide bound, whereby the polycyclic peptide is obtained.

At least one further cyclisation can be a further cyclisation by reacting an amine and an (esterified or thioesterified) carbonyl of the same peptide, which cyclisation can be a head-to-tail cyclisation. The further cyclisation may have preceded the enzymatic cyclisation involving a side-chain reactive group according to the invention, be carried out simultaneously, or be carried out thereafter. The further cyclisation can be an enzymatic cyclisation, but does not have to be.

Alternatively, or in addition a further cyclic structure can be formed by following approach (ii). This can be achieved in a manner known *per se, e.g.* based on the teaching of WO 2005/3414.

### Reaction conditions for enzymatic cyclisation

Reaction conditions can be based known on suitable ligation or cyclisation reaction conditions for the enzyme that is used to catalyse the cyclisation in a method according to the invention in combination with the information disclosed herein.

Preferably the reaction is performed in a buffered fluid. In principle, any buffer is suitable. Good buffers are known to a person skilled in the art. See for instance David Sheehan in Physical Biochemistry, 2nd Ed. Wiley-VCH Verlag GmbH, Weinheim 2009; http ://www.sigmaaldrich.com/life-science/core-bioreagents/ biological-buffers/ learning-center/buffer-calculator.html.

In particular, when using a subtilisin BPN' variant or the like (e.g. described hereinabove and the literature discussing this group of enzymes) having the peptide cyclase activity catalysing a cyclisation in a method according to the invention the cyclisation may be carried out in a fluid comprising water. The water content usually is 10-100 vol %, based on total liquids, preferably 20 vol. % or more, preferably 40 vol. % or more, in particular 50 vol. % or more in particular 60 vol. % or more.

For cyclisation reactions the optimal pH can be different from the ligation reaction wherein two peptide fragments are coupled by fragment condensation for which an enzyme used in accordance with the invention may already be known to be catalytically active. The pH for the cyclisation reaction may be at least 3, in particular at least 4, preferably at least 5. A desired maximum pH is usually less than 11, in particular less than 10, preferably less than 9. Usually the optimal pH for the enzymatic cyclisation reactions is between 5 and 9.

In the method of the invention, it may be advantageous to add additives to the fluid wherein the reaction is carried out to improve the solubility of the precursor peptide or to improve the reaction yield. Such additives may be a salt or an organic molecule, for instance guanidinium hydrochloride, urea, sodium dodecasulphate or Tween.

The reaction may be carried out in a fully aqueous liquid or in a mixture of water and a water mixable co-solvent such as N,N-dimethylformamide (DMF), N-methylpyrrolidinone (NMP), N,N-dimethylacetamide (DMA), dimethylsulphoxide (DMSO), acetonitrile, an ether, such as tetrahydrofuran (THF), 2-methyl-tetrahydrofuran (Me-THF) or 1,2-dimethoxyethane, or a (halogenated) alcohol, such as methanol, ethanol, isopropanol, tert-butanol, 2,2,2-trifluoroethanol (TFE), 1,1,1,3,3,3-hexafluoroisopropanol, or a mixture of these organic solvents. Depending on the stability of the peptide cyclase and the solubility of the precursor peptide used as the substrate, the amount of co-solvent is preferably below 70 vol%, more preferably below 60 vol%, even more preferably below 50 vol%, and most preferably below 40vol%.

In principle the temperature during the enzymatic cyclisation is not critical, as long as a temperature is chosen at which the used peptide cyclase shows sufficient activity and stability. Such a temperature is usually known for the peptide cyclase to be used or can be routinely determined, making use of a known substrate for peptide cyclase under known reaction conditions. Generally, the temperature may be at least -10 °C, in particular at least 0 °C or at least 10 °C. Generally, the temperature may be 70°C or less, in particular 60°C or less or 50 °C or less. Optimal temperature conditions can easily be identified for a specific cyclisation by a person skilled in the art through routine experimentation based on common general knowledge and the information disclosed herein. In general, the temperature advantageously is in the range of 20-50°C.

### Post-cyclisation

A cyclic peptide synthesised in a method according to the invention may contain a chain comprising one or more amino acids, outside the ring structure, e.g. comprising the N-terminus in case of a head-to-side chain synthesis, the C-terminus in case of a tail-to-side chain synthesis, or both as may be the case after a side-chain-to side chain synthesis. Such chain can be useful as a handle to attach the peptide e.g. to a solid phase to immobilise it or to a probe or to another molecule, e.g. a lipid, a carbohydrate, a synthetic polymer, a marker (e.g. fluorescent, phosphorescent, radioactive) or another peptide, e.g. a protein, such asan antibody. If it is undesired, it may be cleaved off from the cyclic structure, e.g. enzymatically.

The invention will now be illustrated by the following examples.

### Example 1 (cyclic peptide comprising an oxvtocine sequence)

### Solid Phase Peptide Synthesis of Ac-DFSKK(CYIQNCPLG)-OCam-LKK:

0.5 gram Rink amide resin (loading 0.62 mmol/gram) was used for synthesis. Unless noted differently dimethylformamide (DMF) is used as solvent. 20 %v/v solution of piperidine in DMF (dimethylformamide) was used to remove Fmoc from the peptide (2 times 10 min). Coupling of the amino acids (4 eq.) was performed with diisopropylcarbodiimide (DIC; 6eq) and OxymaPure (4 eq) (40min); after 20 min a second time DIC (6 eq) was added. 10 ml solvent per gram resin were used standardly. Coupling times for peptides longer than 10 amino acids were increased to 60 minutes respectively. Cam-ester was introduced using Fmoc-glycolic acid according to Nuijens et al. (Engineering a Diverse Ligase Toolbox for Peptide Segment Condensation. (2016), doi:10.1002/adsc.201600774)), see also Toplak, A et al. (Peptiligase , an Enzyme for Efficient Chemoenzymatic Peptide Synthesis and Cyclization in Water. (2016). doi:10.1002/adsc.201600017)

Sidechain functionalization was achieved using Fmoc-Lys(MTT)-OH. Selective removal of MTT protecting group was done using 6,5:2:1:0,5 (v/v/v/v) DCM/HFIP/TFE/TIS. Acetylation was performed using a solution of DMF: N,N-diisopropylethylamine (DIPEA): Acetic Anhydride (10:1:1). Final cleavage was performed using 87.5/5/2.5/2.5/2.5 (v/v/v/v/w) TFA/TIS/H₂O/thioanisole/DTT for 3 hours. Subsequently, the peptide was precipitated in cold diisopropylether (40ml solvent for 10ml TFA) for three times. After lyophilizing, the peptide was purified via preparative HPLC and pure fractions were again lyophilized.

### Purification of Ac-DFSKK(CYIQNCPLG)-OCam-LKK:

The crude product of the precursor peptide was purified using HPLC and the following columns:
Analytical column: Phenomex Kinetex 5 µm EVO C18 100Å LC Column 150 x 4.6 mm Preparative column: XBridge Prep C18 5 µm OBD ^{™} Column 30 x 100 mm

The crude sample of the peptide was dissolved in a solution of 50 % acetonitrile (can) in ultrapure water (milliQ water, MQ) to a concentration around 20 mg/mL. An isocratic gradient of 20-30% of ACN (+ 0,1% TFA) in MQ (+ 0,1% TFA) was used during the purification process.

Analytical LC-MS was used to confirm that the purified precursor peptide was obtained.

### Tail-to-side chain cyclisation Ac-DFSKK(CYIQNCPLG)-OCam-LKK

### Preparation of the phosphate buffer stock solutions

To prepare 2 mL of the phosphate buffer stock solution (200 mmol/L) containing TCEP (1.5 mg/ml), the following reactants were used:
- 400 µL of 1M stock solution of phosphate buffer
- 30 µL. of 100 mg/ml TCEP
- milliQ water

The pH was adjusted using solutions of 3M of NaOH to get the final pH of buffer stock solution of 8.3 at temp 25 °C.

### Preparation of the peptide stock solutions

To prepare the stock solution of peptide in concentration of 0.5 mmol/L, 0.42 mg of the pure precursor peptide was weighed out. Then it was dissolved in 600 µL of buffer stock solution.

The concentration of 0.25 mmol/L was obtained by diluting 300 µL of 0.5 mmol/L peptide solution and 300 µL of buffer stock solution. After diluting the peptide in buffer, the pH was 8.30.

### Cvclisation

The ligations were performed with Omniligase 1 using 0.25 mM peptide in 200 mM potassium phosphate buffer pH 8.2-8.3 containing 1.5 mg/mL TCEP. The detailed reaction procedure is described below.

The 220 µL volume of peptide solution was transferred to the Eppendorf vials. Then 2.2 µL of 5.02 mg/ml Omniligase-1 enzyme was added to each reaction vial.:. The concentration of enzyme the reaction vial was 0.05 mg/ml.

The reaction was monitored by sampling at the following time points: 15, 30, 60 and 120 min. At each time point, 50 µL sample of each reaction mixture was added to 50 µL of quenching solution (MQ:ACN:TFA 48v:48v:4v). The 0 min sample was prepared as a reference point by quenching the peptide stock solution (50 µL) of each concentration in 50 µL of quenching solution before addition of enzyme.

All samples were measured in LC-MS Agilent System using the isocratic gradient of 5-60% of ACN (+ 0,05% FA) in MQ (+ 0,05% FA).
Column: Phenomex Kinetex 5 µm EVO C18 100Å LC Column 150 x 4.6 mm

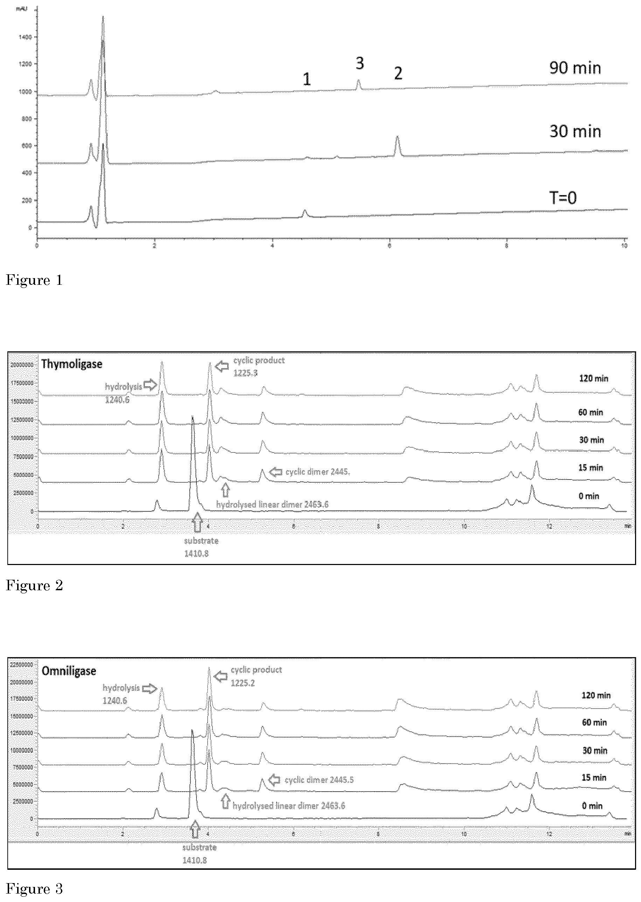

Full conversion of the end to side-chain cyclization of the precursor peptide was obtained. The substrate was fully converted to the cyclic product after 15 min and almost no dimer formation was observed (Figure 1, middle chromatogram, peak 2 shows the product after 30 min).

### Trypsin digestion:

It was found possible to remove the peptide sequence remaining outside the cyclic structure (DFSK) from the obtained cyclic peptide. This was accomplished as follows: Trypsin solution was added to the reaction mixture to a protease to peptide ratio of 1:100 (w/w), 30 min after starting cyclisation. After 1 hour, the trypsin digestion was analyzed using LC/MS and showed full conversion into a cyclic structure consisting of 10 amino acid residues (cyclic (K CYIQNCPLG), peak 3 in top chromatogram of Figure 1)_and the linear tetrapeptide DFSK.

### Example 2: peptide with cyclic structure of six amino acid units

The precursor peptide *Ac-Abz-DFSKK(ALKSG)-OCam-L* was prepared by Solid Phase Peptide Synthesis in accordance with Example 1, Nuijens et al. and Toplak et al.

### Tail to side-chain cyclization of Ac-Abz-DFSKK(ALKSG)-OCam-L

Conditions were as in Example 1, unless specified otherwise.

To prepare the peptide stock solution in concentration of 0.25 mmol/L, 0.46 mg of pure Ac-Abz-DFSKK(ALKSG)-OCam-L was weighed out. Then it was dissolved in 1.30 mL of phosphate buffer stock solution.

After dissolving the peptide in the buffer stock solution, pH 8.30 was recorded.

### Cyclisation process of Ac-Abz-DFSKK(ALKSG)-OCam-L

The cyclisations were performed using 0.25 mM peptide in 200 mM potassium phosphate buffer solution, containing 1.5 mg/mL TCEP, at pH 8.3. The concentration of enzyme in the reaction solution was 0.05mg/mL.

The enzymes used in this experiment were thymoligase and omniligase

The detailed reaction procedure is described below.

220 µL of peptide solution (c=0.25 mM) was transferred to two Eppendorf vials. Thereafter the calculated volumes of the enzyme were added.

The reaction was monitored by sampling at the following time points: 15, 30, 60 and 120 min. At each time point, 50 µL sample of each reaction mixture was added to 50 µL of quenching solution (MQ:ACN:TFA 48v:48v:4v). The 0 min sample was prepared as a reference point by quenching the peptide stock solution (50 µL) of each concentration in 50 µL of quenching solution before addition of enzyme.

All samples were measured in LC-MS Agilent System using the isocratic gradient of 5-60% of ACN (+ 0,05% FA) in MQ (+ 0,05% FA).

Column: Phenomex Kinetex 5 µm EVO C18 100Å LC Column 150 x 4.6 mm

The substrate was fully converted after 15 min of reaction, as illustrated in Figure 2 (thymoligase) and Figure 3 (omniligase). Both used enzymes presented similar activity in performing the cyclization reaction. However, the best results were achieved with omniligase because there was the smallest dimer formation, and the hydrolysis was relatively low.

## Claims

1. Method for enzymatically synthesizing a cyclic peptide, comprising providing a precursor peptide, wherein the precursor peptide comprises a first reactive group, which first reactive group is an unprotected amine group and a second reactive group which second reactive group is a peptide ester or peptide thioester, wherein at least one of said reactive groups is a reactive side-chain or a part of a side-chain of the precursor peptide, and subjecting the precursor peptide to an enzymatic cyclization in the presence of a peptide cyclase, wherein the first reactive group and the second reactive group form an amide bond whereby the cyclic peptide is obtained, with the proviso that at least one of said reactive groups is the reactive side-chain or part of a side-chain of the precursor peptide.

2. Method according to claim 1, wherein the precursor peptide from which the cyclic peptide is to be synthesized is a branched peptide comprising a backbone and a side-chain comprising one or more amino acid units, the side-chain comprising one of said reactive groups participating in the enzymatic cyclization and wherein the reactive group of the side-chain participating in the enzymatic cyclization reacts with either a peptide C-terminal ester or thioester of the peptide or with an unprotected N-terminal amine of the peptide, thereby forming the amide bond.

3. Method according to claim 2, wherein
the branched peptide has a side-chain, which comprises one or more amino acid units, preferably 2-30 amino acid units, in particular 4-20 amino acid units, and which has an unprotected amine at the end of the side chain , which unprotected amine participates in the enzymatic cyclization as the first reactive group, wherein
the branched peptide has a peptide C-terminal ester or thioester, which peptide C-terminal ester or thioester participates in the enzymatic cyclization as the second reactive group, and wherein
said side chain is bound to the backbone at the C-terminal amino acid unit (PI), at the penultimate amino acid unit (P2) next to the C-terminal amino acid unit (PI), or at the amino acid unit (P3) next to the penultimate amino acid.

4. Method according to any of the preceding claims, wherein the amino acid unit of the backbone at which the side-chain providing the unprotected amine participating in the enzymatic cyclisation is attached, is selected from the group consisting of lysine units, ornithine units, 2,4-diamino butanoic acid units and 2,3-diamino propionic acid units.

5. Method according to any of the preceding claims, wherein the peptide C-terminal ester or thioester providing the second reactive group has a chain length of at least four amino acid units and the fourth amino acid (P4) counted from the amino acid at which the peptide ester or thioester is present, is selected from the group consisting of phenylalanine units, valine units, isoleucine units, leucine units, tyrosine units and tryptophan units, preferably from the group consisting of phenylalanine units, valine units, isoleucine units and leucine units.

6. Method according to claim 2, wherein the provided peptide from which the cyclic peptide is to be synthesized is a branched peptide, the branched peptide having an unprotected *N*-terminal amine, which unprotected N-terminal amine participates in the enzymatic cyclization as the first reactive group and wherein the branched peptide has a side-chain which comprises the peptide ester or thioester - typically present at the end of the side chain, counted from where the side-chain is attached to the backbone - participating as the second reactive group in the enzymatic cyclization.

7. Method according to claim 1, wherein the first reactive group is an amine group of a first side-chain of the peptide from which the cyclic peptide is to be synthesized and the second reactive group is an ester or thioester group of a second side-chain of the peptide from which the cyclic peptide is to be synthesized.

8. Method according to any of the preceding claims, wherein a polycyclic peptide is synthesised having at least two cyclic structures comprising one or more peptide bonds.

9. Method according to claim 8, wherein a cyclic peptide is provided having a first side chain comprising the first reactive group and a second side chain comprising the second reactive group and these reactive groups are react with each other thereby forming the amide bound, whereby the polycyclic peptide is obtained.

10. Method according to claim 8 or 9, wherein the enzymatically synthesized cyclic peptide comprises a first and a second methionine unit in the cyclic structure and a disulphide bridge or a methylene bridge is formed between said first and said second methionine unit.

11. Method according to claim any of the preceding claims, wherein a side chain providing said first reactive group or providing said second reactive group comprises at least two amino acid units, preferably 3-30 amino acid units, more preferably 4-20 amino acid units, in particular 5-15 amino acids more in particular 5, 6, 7, 8, 9 or 10 amino acid units.

12. Method according to any of the preceding claims, wherein the precursor peptide comprises a side-chain providing a second reactive group, wherein said side-chain is attached to the peptide backbone at an amino acid unit selected from the group consisting of glutamic acid units, aspartic acid units, 2-aminohexane dioic acid units and 2-aminoheptanedioic acid units.

13. Method according to any of the preceding claims, wherein the precursor peptide comprises a side-chain providing a second reactive group, wherein said side-chain has at least four amino acid units and the fourth amino acid (P4) counted from the amino acid unit at which the peptide ester or thioester participating as the second reactive group is present, is selected from the group consisting of phenylalanine units, valine units, isoleucine units, leucine units, tyrosine units and tryptophan units, preferably from the group consisting of phenylalanine units, valine units, isoleucine units and leucine units.

14. Method according to any of the preceding claims, wherein by cyclisation reaction a cyclic structure is obtained formed by 10 or more covalent bonds, in particular by 12-100 covalent bonds, preferably by 15-60 covalent bonds.

15. Method according to any of the preceding claims wherein the cyclic peptide that is obtained comprises an amino acid or peptide branch that has not participated in the cyclization reaction, and wherein said branch or part thereof is cleaved off from the cyclic structure or wherein at the amino acid unit most remote from the cyclic structure a functional group is present which functional group is reacted with a functional group of a different moiety, which may be a another peptide, another polymeric compound or a solid material.
